# EUROPEAN PATENT SPECIFICATION

(11) **EP 4 466 244 B1**
(45) Date of publication and mention of the grant of the patent: **08.07.2026**
(21) Application number: 23705889.6
(22) Date of filing: 19.01.2023
(51) Int. Cl.: C07C 17/02, C07C 19/045, C07C 17/156

(54) **OXYCHLORINATION PROCESS**
OXYCHLORIERUNGSVERFAHREN
PROCÉDÉ D'OXYCHLORATION

(30) Priority: 19.01.2022 US 202263300799 P
(43) Date of publication of application: 27.11.2024
(73) Proprietor: Oxy Vinyls, LP, Dallas, Texas 75254 (US)
(72) Inventor: KRAMER, Keith S., Andover, Kansas 67002 (US); ZAKZESKI, Joseph, 67056 Ludwigshafen (DE); PARVULESCU, Elena, 67056 Ludwigshafen (DE)
(74) Representative: Gregorj S.r.l.
(86) International application number: PCT/US2023/060871
(87) International publication number: WO 2023/141486

(56) References cited:
- EP-B1- 0 058 644
- EP-B1- 0 058 976
- EP-B1- 0 278 922
- US-A- 4 265 837
- US-A- 4 740 642

## Description

This application claims the benefit of U.S. Provisional Application No. 63/300,799 filed on January 19, 2022.

### FIELD OF THE INVENTION

Embodiments of the present invention provide an oxychlorination process that employs an activated catalyst.

### BACKGROUND OF THE INVENTION

Ethylene dichloride, which is also referred to as 1,2-dichloroethane (EDC), can be produced by the oxychlorination of ethylene. The oxychlorination process converts ethylene to EDC through a reaction involving hydrochloric acid and oxygen (or an oxygen-containing species). Water is produced as a by-product. Both fixed bed and fluidized bed processes have been used commercially. Fluidized bed processes are typically characterized by a more homogeneous temperature distribution throughout the reactor because fluidized beds have enhanced transport characteristics that allow gradual exchange of a catalyst during operation without the need to shut down the reactor.

Industrial oxychlorination processes are typically conducted using excess ethylene, which serves to minimize unreacted hydrochloric acid (HCl). As those skilled in the art appreciate, the presence of HCl in the product stream is detrimental because it is not easily removed and can serve to degrade downstream equipment. HCl conversion is also temperature dependent, with higher temperatures driving higher HCl conversion. As a result, industrial processes typically operate at temperatures greater than 220 °C. While high temperatures result in increased catalytic activity for the oxidation of ethylene to EDC, higher temperatures also unfortunately promote undesirable side reactions that lead to CO and CO₂ formation, as well as chlorinated hydrocarbons. In other words, EDC selectivity, which is a measure of ethylene converted to EDC, decreases with increased concentrations of byproducts in the product stream. Among the chlorinated hydrocarbon byproducts, 1,1,2-trichloroethane, chloral, ethylchloride, chloroform and carbon tetrachloride are common and are often oxidized as a waste stream. Moreover, heavier chlorinated organic byproducts can also be produced, and therefore not only reduce EDC selectivity but also frustrate the product purity since the heavy chlorinated organic byproducts are not easily separated.

It is therefore desirable to reduce the operating temperature of any given catalytic system without reducing HCl conversion and thereby gain EDC selectivity without a commensurate loss in process efficiency.

### SUMMARY OF THE INVENTION

One or more embodiments of the present invention provide a method for the production of 1,2-dichloroethane, the method comprising (i) providing a fluidizable catalyst; (ii) charging the fluidizable catalyst to a fluidized bed reactor; (iii) fluidizing the catalyst within the reactor; (iv) heating the catalyst to a temperature of greater than 210 °C for greater than 24 hours within the fluidized bed reactor while fluidizing the catalyst within the reactor, where said steps of fluidizing and heating take place in the absence of the conversion of ethylene; and (v) after said step of heating, converting ethylene to 1,2-dirchloroethane in the presence of the catalyst.

### DETAILED DESCRIPTION OF ILLUSTRATIVE EMBODIMENTS

Embodiments of the invention are based, at least in part, on the discovery of an oxychlorination process that takes place within a fluidized bed in the presence of an activated catalyst. According to aspects of the invention, the catalyst is activated prior to the conversion of ethylene to 1,2-dichloroethane. In one or more embodiments, activation generally includes heating the catalyst over a sufficient period of time under fluidization conditions in the absence of ethylene conversion. It has been observed that by activating the catalyst, the amount of ethylene converted to 1,2-dichloroethane (i.e. EDC selectivity), relative to other conversion products, is increased without a negative impact on process efficiency. Stated differently, activation of the catalyst allows for efficient 1,2-dichloroethane production while producing fewer undesirable by-products.

### PROCESS OVERVIEW AND REACTOR CONFIGURATION

In one or more embodiments, the oxychlorination process of the present invention takes place within a fluidized bed reactor, which is a reactor adapted to hold and fluidize a catalyst. Fluidized bed reactors and processes for their use in oxychlorination of ethylene to 1,2-dichloroethane (EDC) are generally known as disclosed in U.S. Patent No. 5,292,703 and U.S. Publication Nos. 2009/0054708, 2009/0298682, 2010/0274061, 2006/0129008, 2004/0192978 and US4740642. In one or more embodiments, the fluidized bed reactor includes a reaction zone in which the conversion of ethylene to EDC takes place. In one or more embodiments, the reactor is equipped with temperature control elements, such as steam coils, that can be used to either heat or cool the reactor, particularly the reaction zone.

In one or more embodiments, at least a portion of the fluidized bed reactor includes a fluidizable catalyst composite. The catalyst composite, which may simply be referred to as a catalyst, includes catalytic materials impregnated onto a solid support. The catalytic materials, which are typically catalytic metal compounds, and the solid support are described in greater detail below.

In one or more embodiments, ethylene, hydrochloric acid, oxygen gas and/or oxygen-containing species, and optionally inert gas is fed to the reactor as one or more fluid streams. These fluid streams may be injected into the reactor under conditions, such as force and/or velocity, to cause the catalyst composite to fluidize within the bed. In other words, as the skilled person appreciates, the fluid stream(s) are passed through the reactor (e.g. the reaction zone) in a manner that suspends and mixes the catalyst composites and causes them to behave in a fashion similar to a fluid. In one or more embodiments, the reactant and/or gas feeds are fed into the bottom of the reactor and enter the reaction zone through one or more distribution elements. In one or more embodiments, the product stream, which includes EDC, ethylene by-products, water, unreacted reactant, and optionally inert gas, exits the reactor through an exit port located at or near the top of the reactor.

In one or more embodiments, the reactor is configured for a once-through process whereby any unreacted ethylene within the product stream is vented or otherwise removed. Alternatively, the reactor is configured for a recycle process whereby the unreacted ethylene is recycled back into the reactor. In one or more embodiments, the product stream undergoes separation to separate non-condensable constituents, liquid organics, and water-soluble compounds. For example, the product stream may be directed to a quenching system that condenses EDC and hydrochloric acid to form a gaseous non-condensable stream and a liquid stream. The liquid stream may undergo a liquid-liquid separation to isolate the EDC from the aqueous constituents. The non-condensable stream, which can include ethylene, oxygen, and nitrogen, as well as other gaseous constituents, can be recycled back into the reactor.

### CATALYST COMPOSITE

As suggested above, the catalyst, which may be referred to as a catalyst composite or catalyst particle, includes a support that is impregnated with a catalytic metal compound; i.e. a support with a catalytic metal compound deposited thereon. In one or more embodiments, the catalytic metal compound includes a copper compound optionally together with one or more complementary catalytic metals compounds, which may also be referred to as promoters. Catalysts that are useful for oxychlorination are generally known as described in, for example, U.S. Publication No. 2014/0128643 and U.S. Patent No. 5,292,703, EP0278922 and EP0058644.

### SUPPORT MATERIALS

In one or more embodiments, the support, which is generally a fluidizable high-surface area support material, may be prepared from silica, magnesia, kieselguhr, clay, fuller's earth, alumina, zeolites or combinations thereof. According one or more embodiments, the support can be a high surface area powder (e.g. gamma, delta or theta alumina powder) including particles, granules and/or spheres (e.g. alumina microspheres or nanospheres in amorphous or colloidal form). In embodiments, alumina supports can be activated or transition aluminas generated by calcination of a hydrated or hydroxylated precursor alumina. These activated aluminas can be identified by their ordered structures observable by their X-ray diffraction patterns, which indicate a mixed phase material containing minimal to no low-surface area or crystalline phase alpha alumina.

The skilled person appreciates that alpha alumina can be identified by a defined crystalline phase by x-ray diffraction. The higher surface area activated aluminas are often defined as a gamma alumina phase, but the phase transitions can be a continuum of varying percentages of multiple mixed phases such as, but not limited to, delta and theta phases based on the chosen calcination temperature to achieve the desired support surface area. In particular embodiments, gamma alumina is employed as a support. The alumina supports (e.g. gamma alumina powder) may be referred to as fluidizable alumina supports or fluidizable alumina support materials.

In one or more embodiments, useful gamma alumina supports are powders having a gamma phase of at least 90%, or at least 95%, or at least 98%, or at least 99%, or at least 99.995%. In these or other embodiments, useful gamma alumina supports have a surface area of about 50 m²/g to about 250 m²/g, or about 70 m²/g to about 225 m²/g, or about 100 m²/g to about 215 m²/g, or about 150 m²/g to about 205 m²/g. According to embodiments, useful alumina supports (e.g., gamma alumina powder) have a surface area of at least 80 m²/g. In these or other embodiments, useful fluidizable alumina supports may have a compacted bulk density of about 0.5 g/cm³ to about 4.0 g/cm³, or about 0.75 g/cm³ to about 3.0 cm³, or about 1.0 cm³ to about 2.0 cm³, or about 0.7 cm³ to about 1.3 cm³, or about 3.65 cm³. In these or other embodiments, useful alumina support materials can have a pore volume of about 0.1 cm³/g to about 2 cm³/g, or about 0.2 cm³/g to about 1 cm³/g, or about 0.3 cm³/g to about 0.75 cm³/g. In these or other embodiments, the fluidizable alumina support materials can have a particle size distribution such that (a) about 90 to about 100 percent by volume of the particles are less than about 150 µm, or less than about 140 µm, or less than about 125 µm, or less than about 110 µm in diameter; (b) about 50 to about 60 percent by volume of the particles are less than about 75 µm, or less than about 70 µm, or less than about 65 µm, or less than about 60 µm; and (c) about 20 to about 35 percent by volume of the particles are less than about 45 µm, or less than about 40 µm, or less than about 35 µm, less than about 30 µm, as measured by a Malvern Instruments, Ltd. Fraunhofer laser diffraction (i.e. light scattering) analyzer where, at room temperature (about 20 °C to about 25 °C) and pressure (about 101325 Pa (1 atm)), 5 g samples of the material are dispersed in 100 mL of deionized water with a magnetic stirrer for 1 minute to form a suspension and 4 mL of the suspension is diluted with 130 mL of deionized water and analyzed at 2500 rotations/minute with the analyzer (at room temperature and pressure).

Useful alumina supports are commercially available. For example, useful supports can be obtained under the tradename Puralox^{®} SCCa-57/170 and Catalox^{®} SCCa-25/200 (Sasol). These aluminas are high-purity activated aluminas that are readily fluidizable, relatively stable, mechanically strong, and resistant to attrition.

In some non-limiting embodiments, the alumina supports may contain additional components. Examples of these additional components include, but are not limited to, lanthanum (La), cerium (Ce), titanium (Ti), silicon (Si), etc. as trace components dispersed throughout the alumina support prior to impregnation of the active catalyst formulation. For example, the alumina support can include about 0.005 wt % to about 0.50 wt % of one or more of the additional components.

It is also recognized that some alumina support materials may contain, in addition to aluminum oxide (Al₂O₃) (and additional components), small amounts of impurities of other metals such as metal oxides, for example, less than about 0.02 wt % of sodium oxide, less than about 0.05 wt % of iron oxide (Fe₂O₃), less than about 0.3 wt % of titanium dioxide, less than about 0.2 wt % of silicon dioxide, etc. Also, it is known that Ti compounds may remain in the alumina from the alumina production process.

### CATALYTIC MATERIALS

As suggested above, the catalyst composite typically includes one or more metal compounds. In one or more embodiments, the metal compounds include copper compounds in combination with one or more complementary metal compounds (i.e. promoters). For example, in one or more embodiments, the metal compounds, which may also be referred to as catalytic metal compounds, may include a copper compound, an alkaline earth metal compound (e.g. a magnesium compound), optionally an alkali metal compound (e.g. a potassium compound), and a transition metal compound (e.g. a manganese compound or rhenium compound). Other exemplary catalytic metal compound combinations may include a copper compound, an alkaline earth metal compound (e.g. a magnesium compound), optionally an alkali metal compound (e.g. potassium compound), and a rare earth metal compound (e.g. a lanthanum compound). Still other exemplary catalytic metal compound combinations include a copper compound, an alkaline earth metal compound (e.g. a magnesium compound), and optionally an alkali metal compound (e.g. potassium compound). Other exemplary catalytic metal compound combinations may include a copper compound, an alkaline earth metal compound (e.g. a magnesium compound), optionally an alkali metal compound (e.g. potassium compound), and a zirconium compound (e.g. zirconium oxychloride).

In one or more embodiments, the copper compound is a water-soluble salt such as a copper chloride (e.g. copper II chloride). Other copper salts that can convert to the chloride during the oxychlorination process can also be used, such as the nitrate salt, the carbonate salt, the sulfate salt, or other halide salts like the bromide salts, or combinations thereof.

In one or more embodiments, useful alkali metal compounds include water-soluble salts such as alkali metal chlorides. Other alkali metal salts that can convert to the chloride salt during the oxychlorination process can also be used, such as the nitrate salts, the carbonate salts, the sulfate salts, or other halide salts such as bromide salts, or combinations thereof. In particular embodiments, the alkali metal compound includes a potassium salt such as potassium chloride, potassium nitrate, potassium bromate, potassium carbonate, or potassium sulfate. In specific embodiments, the alkali metal compound is potassium chloride. In one or more embodiments, the alkali metal compound may include potassium chloride and one or more additional alkali metal compounds such as lithium chloride. In other embodiments, the catalytic metal compounds include potassium compounds and either no or only trace amounts (e.g. less than 1 ppm by weight) of other alkali metal compounds.

In one or more embodiments, useful alkaline earth metal compounds include water-soluble salts such as alkaline earth metal chlorides. Other alkaline earth metal salts that can convert to the chloride salt during the oxychlorination process can also be used, such as the nitrate salt, the carbonate salt, the sulfate salt or other halide salts such as the bromide salts, or combinations thereof. In particular embodiments, the alkaline earth metal compound includes a magnesium salt such as magnesium chloride, magnesium nitrate, magnesium bromate, magnesium carbonate, or magnesium sulfate. In specific embodiments, the alkaline earth metal compound is magnesium chloride. In one or more embodiments, the alkaline earth metal compound may include a magnesium compound and one or more additional alkaline earth metal compounds such as compounds of calcium, strontium, barium, or a mixture thereof. In particular embodiments, the catalytic metal compounds include magnesium compounds and either no or only trace amounts (e.g. less than 1 ppm by weight) of other alkaline earth metal compounds.

In one or more embodiments, useful transition metal compounds include water-soluble salts such as transition metal chlorides (e.g. a chloride or an oxychloride). Other salts that can convert to the chloride salt during the oxychlorination process can also be used, such as the nitrate salts, the carbonate salts, the sulfate salts or other halide salts such as the bromide salts, or combinations thereof. Useful transition metal compounds include manganese compounds such as manganese chloride and/or rhenium compounds. Still other useful transition metal compounds include zirconium compounds such as zirconium oxychloride. In one or more embodiments, the catalytic metal compounds may include a transition metal compound in combination with one or more additional transition metal compounds. In other embodiments, the catalytic metal compounds include a transition metal compound (e.g. zirconium oxychloride) and either no or only trace amounts (e.g. less than 0.005 wt %) of other transition metal compounds. In one or more embodiments, the catalytic metal compounds include zirconium and do not contain manganese (Mn) or rhenium (Re).

In one or more embodiments, useful rare earth metal compounds include water-soluble salts such as rare earth metal halides (e.g. a chloride). Other salts that can convert to the chloride salt during the oxychlorination process can also be used, such as nitrate salts, carbonate salts, the sulfate salts or other halide salts such as bromide salts. Useful rare earth metal compounds include lanthanum compounds such as lanthanum chloride. Other useful rare earth metal compounds include cerium, neodymium, and praseodymium compounds. In one or more embodiments, the catalytic metal compounds may include a rare earth metal compound in combination with one or more additional rare earth metal compounds. In other embodiments, the catalytic metal compounds include a rare earth metal compound and either no or only trace amounts (e.g. less than 0.005 wt %) of other rare earth metal compounds. In one or more embodiments, the catalytic metal compounds include a lanthanide metal and does not contain manganese (Mn) or rhenium (Re).

### CATALYTIC METAL LOADINGS

As the skilled person appreciates, the amount of copper metal deposited is generally based on the activity desired and the specific fluidization characteristics of the support for fluid bed catalyst applications. In one or more embodiments, the catalyst composites include from about 1 wt % to about 10 wt % of copper metal based on the total weight of the catalyst composition. In one or more embodiments, the catalyst includes greater than 2.0 wt %, or in other embodiments greater than 3.0 wt % copper (atomic copper) based on the total weight of the catalyst. In these or other embodiments, the catalyst includes less than 6.0 wt %, or in other embodiments less than 8.0 wt %, or in other embodiments less than 10 wt % copper based on the total weight of the catalyst.

Where the catalyst includes an alkali metal compound, the catalyst composite may include from about 0.5 wt % to about 5.0 wt %, or about 0.75 wt % to about 4.0 wt %, or about 1.0 wt %, to about 3.0 wt % alkali metal (e.g. atomic potassium) based on the total weight of the catalyst composition. In one or more embodiments, the catalyst includes greater than 0.5 wt %, or in other embodiments greater than 0.75 wt % alkali metal based on the total weight of the catalyst. In one or more embodiments, the catalyst includes less than 5.0 wt %, or less than 4.0 wt %, or less than 3.0 wt % alkali metal (e.g. potassium metal) based on the total weight of the catalyst composition. If an additional alkali metal is included, the catalyst can contain about 0.005 wt % to about 10.0 wt % of the additional alkali metal.

Where the catalyst includes an alkaline earth metal compound, the catalyst composite may include from about 0.5 wt % to about 5.0 wt %, or about 0.75 wt % to about 4.0 wt %, or about 1.0 wt %, to about 3.0 wt % alkaline earth metal (e.g. atomic magnesium) based on the total weight of the catalyst composition. In one or more embodiments, the catalyst includes greater than 0.5 wt %, or in other embodiments greater than 0.75 wt % alkaline earth metal based on the total weight of the catalyst. In one or more embodiments, the catalyst includes less than 5.0 wt %, or less than 4.0 wt %, or less than 3.0 wt % alkaline earth metal (e.g. magnesium) based on the total weight of the catalyst composition. If an additional alkaline earth metal is included, the catalyst can contain about 0.005 wt % to about 10.0 wt % of the additional alkaline earth metal.

Where the catalyst includes a transition metal compound, the catalyst composite may include from about 0.1 wt % to about 3.0 wt %, or about 0.3 wt % to about 2.5 wt %, or about 0.5 wt %, to about 2.0 wt % transition metal (e.g. atomic manganese) based on the total weight of the catalyst composition. In one or more embodiments, the catalyst includes greater than 0.1 wt %, or in other embodiments greater than 0.3 wt % transition metal based on the total weight of the catalyst. In one or more embodiments, the catalyst includes less than 3.0 wt %, or less than 4.0 wt %, or less than 2.0 wt % transition metal (e.g. manganese metal) based on the total weight of the catalyst composition. If an additional transition metal is included, the catalyst can contain about 0.005 wt % to about 10.0 wt % of the additional transition metal.

Where the catalyst includes a rare earth metal compound, the catalyst composite may include from about 0.1 wt % to about 3.0 wt %, or about 0.3 wt % to about 2.5 wt %, or about 0.5 wt %, to about 2.0 wt % rare earth metal (e.g. atomic lanthanum) based on the total weight of the catalyst composition. In one or more embodiments, the catalyst includes greater than 0.1 wt %, or in other embodiments greater than 0.3 wt % rare earth metal based on the total weight of the catalyst. In one or more embodiments, the catalyst includes less than 3.0 wt %, or less than 4.0 wt %, or less than 2.0 wt % rare earth metal (e.g. lanthanum metal) based on the total weight of the catalyst composition. In one or more embodiments, additional rare earth metals may be included, and the catalyst can contain about 0.005 wt % to about 10.0 wt % of the additional transition metal.

In particular embodiments, the catalyst includes a zirconium salt such as zirconium oxychloride, zirconium carbonate, or zirconium hydroxide. The catalyst can include the zirconium as metal in the range about 0.1 wt % to about 3.0 wt %, or about 0.5 wt % to about 2.0 wt %, or about 0.75 wt % to about 1.0 wt % based on the total weight of the catalyst composition. According to embodiments, the catalyst can include zirconium metal in an amount of less than 3.0 wt %, or less than 2.0 wt %, or less than 1.0 wt % based on the total weight of the catalyst composition. If an additional transition metal is included, the catalyst can contain about 0.005 wt % to about 10.0 wt % of the additional transition metal.

As the skilled person will appreciate, the final catalyst composition containing the catalytic metals (e.g. copper compound, alkaline earth metal compound, and transition metal compound) is readily fluidizable. In one or more embodiments, any other metals can be present in the catalyst compositions in relatively small or trace amounts. Typically, these metals, if present, may be present in amounts of less than about 0.005 wt % based on the total weight of the catalyst composition.

### PREPARATION OF CATALYST COMPOSITE

The catalysts employed in the invention may be prepared by conventional techniques.

For example, the metal compounds can be impregnated onto the alumina support. This can be accomplished by introducing an aqueous solution of the water soluble salts (i.e. metal compounds) to the support. The wetted support can then be dried and optionally calcined. The support can be impregnated with the metal compounds by using a single solution, or in other embodiments, multiple solutions can be used in series. For example, the alkaline earth metal compound, transition metal compound, and any additional metal compounds can be first introduced (and optionally dried and calcined) and then the copper compound can be introduced to the support previously impregnated with the other metal compounds. In this regard, U.S. Publication Nos. 2014/0275661 and 2017/0137351 are referred to.

As the skilled person will appreciate, the impregnation solution can be prepared by dissolving the desired quantity of metal salt in water while stirring (e.g. hand stirring or vigorous mixing) at room temperature or optionally at an elevated temperature (e.g. about 40 °C to about 60 °C). Once dissolved, the metal salt solution can be introduced to the alumina support (i.e. the support is impregnated). For example, the solution can be slowly introduced to a tumbler containing the support (e.g. rotating at about 90 rotations per minute). The salt solution is dosed dropwise or sprayed onto the carrier within 30 minutes. After the impregnation, the wet catalyst is typically dried. This may include pre-drying, which can take place by heating the catalyst over a steam bath (e.g. the catalyst contained in a ceramic dish or other receptacle can be optionally heated over a steam bath for about 1 to about 3 hours). Whether or not pre-dried, the catalyst can be heated in an oven to drive off all or substantially all of the water. For example, the catalyst can be heated in an oven having an environmental temperature of about 100 °C to about 180 °C, or about 110 °C to about 150 °C, or about 100 °C, or about 105 °C, or about 110 °C, or about 115 °C. During this heating step, the catalyst can be exposed to these elevated temperatures for about 2 hours to about 30 hours, or about 10 hours to about 28 hours, or about 20 hours to about 25 hours. After drying, the dried catalyst can be sieved (e.g. sieved hot) by using, for example, a 500 µm sieve, or a 475 µm sieve, or a 450 µm sieve, or a 400 µm sieve, or a 350 µm sieve, or a 300 µm. The dried catalyst can be sieved hot at a temperature of about 100 °C to about 200 °C, or a temperature of 130 °C. The final catalyst material may be packaged (e.g., bottled).

The skilled person appreciates that the specific characteristics of the catalyst, such as surface area and pore volume, can be modified via the deposition of the metal salts. For example, the catalyst compositions as described herein can have a final surface area in the range of about 20 to about 220 m²/g, or about 60 to about 180 m²/g, or about 70 to about 170 m²/g, or about 80 m²/g to about 150 m²/g, or about 150 m²/g to about 215 m²/g.

### CATALYST ACTIVATION

As suggested above, the catalyst compositions described herein can be activated. In one or more embodiments, the catalyst is activated within a reactor bed while being heated and fluidized with a fluidizing gas including one or more of air, oxygen enriched air, nitrogen enriched air, nitrogen, and a combination thereof.

In practicing the methods of one or more embodiments of the present invention, the catalyst is placed into the reactor (i.e. a fluidized bed reactor), which step may be referred to as charging the reactor with the catalyst. After the catalyst is charged to the reactor, activation of the catalyst takes place. In one or more embodiments, activation of the catalyst includes heating the catalyst so that the catalyst composite reaches a threshold temperature for a threshold amount of time under fluidization conditions (i.e. while the catalyst is fluidized). The catalyst is heated to a temperature of greater than 210 °C. Within these embodiments, the temperature of the catalyst is maintained (under fluidization conditions) for greater than 24 hours, and in other embodiments greater than 36 hours.

As indicated above, activation takes place in the substantial absence of ethylene conversion or EDC production. According to aspects of the invention, activation of the catalyst takes place in the substantial absence of hydrochloric acid, which is an amount or less that does not have appreciable impact on the practice of the invention. In one or more embodiments, activation of the catalyst takes place in the presence of less than 100 ppm, in other embodiments less than 50 ppm, and in other embodiments less than 25 ppm hydrochloric acid. In these or other embodiments, activation of the catalyst takes place in the substantial absence of ethylene, which is an amount or less that does not have appreciable impact on the practice of the invention. In one or more embodiments, activation of the catalyst takes place in the presence of less than 100 ppm, in other embodiments less than 50 ppm, and in other embodiments less than 25 ppm ethylene.

In one or more embodiments, catalyst activation takes place while the catalyst is fluidized in the presence of a fluidizing gas stream that includes oxygen gas or an oxygen-containing gas. In these or other embodiments, catalyst activation takes place while the catalyst is fluidized in the presence of fluidizing gas stream that includes inert gas such as argon or nitrogen. In particular embodiments, catalyst activation takes place while the catalyst is fluidized in the presence of a fluidizing gas stream that includes inert gas and is substantially devoid of oxygen or oxygen-containing gas.

As the skilled person appreciates, the catalyst can be heated within the reactor using conventional techniques that may be affected through the temperature control elements of the reactor such as coils or tubes that include heat transfer fluid. Within the reactor, the temperature of the catalyst can be determined by one or more thermocouples positioned within thermal wells located within the fluidized bed, which wells are in contact with the fluidized mass. For purposes of these measurements according to embodiments herein, the fluidized bed, on a macro level, is assumed to be equilibrated and therefore the temperature of the fluidized mass (including gas and solid phases) at the location of the thermal wells is assumed to be at or be approximate to the temperature of the catalyst composite (i.e. the particles). Likewise, the catalyst may be fluidized using conventional techniques that may be affected by injecting pressurized gaseous streams into the reactor at conditions adapted to fluidize the catalyst.

In one or more embodiments, after activation of the catalyst within the reactor, the oxychlorination process is initiated prior to a deleterious change in the nature of the catalyst. As the skilled person will appreciate, initiation of the oxychlorination process takes place by feeding ethylene, hydrochloric acid, and oxygen or oxygen-containing species to the reactor (i.e. the reactant feeds are fed to the reactor). In one or more embodiments, the reactant feeds are fed to the reactor without discontinuing fluidization of the catalyst. In these or other embodiments, the reactant feeds are fed to the reactor prior to the catalyst cooling (after activation) by greater than 50 °C, in other embodiments by greater than 35 °C, in other embodiments by greater than 20 °C, and in other embodiments by greater than 10 °C. In these or other embodiments, the reactant feeds are fed to the reactor prior to the catalyst cooling to a temperature below 110 °C, in other embodiments below 120 °C, in other embodiments below 130 °C, and in other embodiments below 140 °C.

In one or more embodiments, activation of the catalyst takes place prior to using the catalyst in an oxychlorination process. In other embodiments, the catalyst is activated following its use within an oxychlorination process. For example, an oxychlorination process that employs a particular catalyst is paused and the catalyst is activated according the present invention. In one or more embodiments, the reaction is paused by discontinuing at least the hydrochloric acid feed. In other embodiments, the reaction is paused by discontinuing both the hydrochloric acid feed and the ethylene feed.

Without being bound by any particular theory, it is believed that activation of the catalyst according to the present invention removes excess waters of hydration as well as some chlorine, for example, hydrochloric acid, content from the catalysts. As such, this activation process may also modify the surface chemistry of the catalyst, making it more effective in certain applications. While embodiments of the invention require that the catalyst be fluidized during activation and that conditions of heat and fluidization be maintained until the oxychlorination process is started, it is believed that perhaps activation of the catalyst can take place outside of the reactor so long as the catalyst is maintained in a water-free environment up until the oxychlorination process is initiated. For example, it is believed that the benefits of the invention can be achieved if the activated catalyst is maintained under anaerobic conditions such as nitrogen purge or nitrogen blanket during storage, shipment, and charging into the reactor. It is also believed that the benefits of the invention can be achieved if the catalyst is maintained at threshold temperatures during storage, shipment, and charging into the reactor.

### OXYCHLORINATION PROCESS

The catalyst compositions of the invention are highly efficient catalysts for the oxychlorination of ethylene to EDC. The reaction process temperatures vary from about 170 °C to about 260 °C, in other embodiments from about 180 °C to about 250 °C, and in other embodiments from about 190 °C to about 240 °C. Reaction pressures vary from atmospheric to as high as about 1378951.456 Pa (200 psig). Contact times in the fluid bed and fixed bed catalysis can vary from about 5 seconds to about 50 seconds (contact time is defined here as the ratio of reactor volume taken up by the catalyst to the volumetric flow rate of the feed gases at the reactor control temperature and top pressure), and in other embodiments from about 5 seconds to about 35 seconds. The ratio of the ethylene, HCl, and oxygen reactants, based on the moles of HCl fed to the reactor, may range from about 1.0 to about 2.0 moles of ethylene and about 0.5 to about 0.9 mole of oxygen per 2.0 moles of HCl. As previously mentioned, modern oxychlorination processes attempt to operate within the stoichiometric ratio of about 1 to about 2 moles of HCl to 1 mole of ethylene.

As suggested above, the oxychlorination processes of this invention convert ethylene to 1,2-dichloroethane (EDC) in the presence of an activated catalyst. Generally, this reaction proceeds in the presence of two moles of hydrochloric acid and a half mole of oxygen to produce EDC and water. In one or more embodiments, the oxychlorination catalyst of this invention can advantageously be used in oxychlorination processes where the molar ratio of oxygen to hydrogen chloride (O₂/2HCl) approaches a stoichiometric feed rate of 0.5. In one or more embodiments, the process operates at a molar ratio of oxygen to hydrogen chloride (O₂/2HCl) of less than 0.9, in other embodiments less than 0.64, in other embodiments less than 0.58, in other embodiments less than 0.55, and in other embodiments less than 0.52. In one or more embodiments, the process operates at a molar ratio of oxygen to hydrogen chloride (O₂/2HCl) of from about 0.50 to about 0.80, in other embodiments from about 0.51 to about 0.70, in other embodiments from about 0.52 to about 0.60, and in other embodiments from about 0.53 to about 0.58.

In those embodiments where recycle is conducted, the ratio of HCl to ethylene may be lower than 2. In those embodiments where a once-through process is conducted, the ratio of HCl to ethylene may be higher and can approach or be closer to 2, which results in an overall HCl to ethylene molar operating range of about 1 to about 2.

The oxychlorination processes described herein can be operated at or above conventional operating temperatures, such as 220 °C. In other embodiments, the oxychlorination process can be operated at temperatures of less than 218 °C, in other embodiments less than 215 °C, or in other embodiments less than 212 °C. In one or more embodiments, the oxychlorination process of the present invention takes place at temperatures of from about 180 to 220 °C, in other embodiments from about 190 to about 218 °C, in other embodiments from about 195 to about 215 °C, and in other embodiments from about 200 to about 210 °C.

In these or other embodiments, the reaction pressures can vary from about atmospheric to as high as about 1378951.456 (200 psig). Contact times in fluid bed and fixed bed catalysis can vary from about 10 seconds to about 50 seconds (contact time is defined as the ratio of reactor volume taken up by the catalyst to the volumetric flow rate of the feed gases at the reactor control temperature and top pressure), and can be from about 20 seconds to about 35 seconds. The ratio of the ethylene, HCl, and oxygen reactants, based on the moles of HCl fed to the reactor, can range from about 1.0 moles to about 2.0 moles of ethylene and about 0.5 mole to about 0.9 mole of oxygen per 2.0 moles of HCl. Typical oxychlorination processes attempt to operate within the stoichiometric ratio of about 1 mole to about 2 moles of HCl to 1 mole of ethylene.

### CHARACTERISTICS OF PROCESS

The process of the present invention advantageously provides for the efficient conversion of ethylene to EDC. As the skilled person will appreciate, conversion to EDC can be measured by conversion of hydrochloric acid. According to aspects of the present invention, activation of the catalyst according the embodiments described herein results in improvements in EDC yield of greater than 0.1%, in other embodiments greater than 0.25%, in other embodiments greater than 0.5%, and in other embodiments greater than 0.75% when the oxychlorination takes place at temperatures of greater than 220 °C. In other embodiments, activation of the catalyst according the embodiments described herein results in improvements in EDC yield of greater than 1.0%, in other embodiments greater than 1.25%, in other embodiments greater than 1.5%, and in other embodiments greater than 2.0% when the oxychlorination takes place at temperatures of from about 200 °C to 220 °C.

### EXAMPLES

In order to demonstrate the practice of the present invention, the following examples have been prepared and tested. The examples should not, however, be viewed as limiting the scope of the invention. The claims will serve to define the invention.

A series of laboratory-scale, continuous oxychlorination reactions (i.e. runs) were performed. The equipment and procedures employed for each run were generally identical except as outlined below. Likewise, each catalyst was similarly prepared except as provided below.

The catalyst employed in each run was prepared by impregnating an alumina support with a catalyst impregnation solution. The alumina support was obtained under the tradename SCCa 25/200 (Sasol), and was characterized by a pore volume of 0.44 mL/g and a surface area of 203 m²/g. The particle size distribution (psd) of the support was characterized in that 1.4% of the particles were smaller than 22 microns, 7.1% of the particles were smaller than 31 microns, 23% of the particles were smaller than 44 microns, 79% were smaller than 88 microns, and 94% were smaller than 125 microns.

The catalyst impregnation solution was prepared by dissolving metal chlorides in water, and then the solution was distributed evenly over the entire support. The volume of the solution was chosen to match 95% +/- 3% of the pore volume of the support. The impregnated catalyst was then dried at a temperature up to 150 °C to remove most of the water prior to oven drying for an additional 16 hours at 110 °C. The metals employed, as well as the amount of each metal employed for each run, are set forth in the following Tables. For clarity, the metals are provided in weight %, which represents the weight of each metal relative to the entire catalyst composition (e.g. weight percent of copper, as a metal, relative to the total weight of the catalyst composition, which includes all the metal chlorides and the total weight of the support).

While multiple reactors were employed, each reactor had an internal diameter of about 2 cm, and they were filled with catalyst to achieve a fluidized bed height of about 99 +/- 2.5 cm. The reactors were equipped with a centered thermocouple in the fluidized bed and an external electric heater to maintain the reaction temperature at the desired temperature. Each reactor was configured to communicate with analytical equipment adapted to measure HCl in the feed and product stream, and N₂, C₂H₄, O₂, COx, and chlorinated hydrocarbons in the product stream. Specifically, HCl in the feed and product gas was measured via titration, and N₂, C₂H₄, O₂, COx and chlorinated hydrocarbons were measured via GC (HP 6890 Series; Column types--1) Vocol glass capillary column (60 meter; 0.75 mm ID; 1.5 micron film thickness. 2) 80/100 Porapak N column (30.48 x 0.3175 cm (12 x 1/8 in), stainless steel). 3) 60/80 molecular sieve, 5 angstrom (182.88 x 0.3175 cm (6 foot x 1/8 inch)); Detectors--2 TCD's. Detector B (Vocal column) Detector A (mol sieve/Porapak); One TCD is used to detect light gases, such as O₂, N₂, and CO from the molecular sieve column, and heavier gases, such as CO₂ and ethylene as well as lighter chlorinated hydrocarbons such as vinyl chloride and ethyl chloride from the Porapak column. The second TCD is used to detect the remaining heavier chlorinated hydrocarbons from the Vocol column starting with chloroform, including EDC and other heavier chlorinated by-products.

During oxychlorination, the reactors were operated at atmospheric pressure over a temperature continuum with a feed-gas stream composed of 11.4 NL/h of N₂, 3.75 NL/h of ethylene, 7.12 NL/h of HCl and 2.55 NL/h of O₂, unless otherwise specified in the following Tables. Data was collected over the temperature continuum and interpolated over the temperature range. Specifically, after reaction conditions were equilibrated at any given set temperature, data was collected relative to EDC selectivity (moles of ethylene converted to EDC relative to total moles of ethylene converted) and HCl conversion. The temperature of the reaction was then increased (generally about 3-5 °C) and allowed to equilibrate, and data was again collected relative to EDC selectivity and HCl conversion. Once the data was interpolated, the reactions were compared (based upon HCl conversion) at the same performance levels of 98% and 97% selectivity of ethylene to EDC. These comparisons are set forth in following Tables.

Relevant to the present invention, runs were made with and without catalyst activation. Generally speaking, those catalysts that were activated underwent fluidization at an elevated temperature with a feed-gas stream composed of nitrogen and oxygen (unless specified) at ratios and for a time as specified in the following Tables. For those runs that were not activated, the columns were loaded with the catalyst and the reactors were fluidized with nitrogen and brought to temperature, which was about 200 °C. Within no more than about 2 hours of reaching the targeted temperature, the ethylene, oxygen, and HCl feeds were started and the oxychlorination reaction proceeded.

A nonactivated catalyst runs hotter and at lower HCl conversion to achieve the same EDC selectivity as an activated catalyst. Activation is demonstrated by the catalyst showing higher HCl conversion at a lower operating temperature to achieve the same EDC selectivity. Overall performance improves because the non-activated catalyst would need to be operated at a higher temperature to achieve the same HCl conversion as the activated catalyst thus resulting in lower percentage selectivity to EDC.

### Data Set I - Runs 1-6

A first data set, which is summarized in Table I below, generally shows the impact of catalyst activation combined with the impact of catalyst metal ratios.

As set forth in Table I, Runs 1 and 2, Runs 3 and 4, and Runs 5 and 6 each respectively used the same the catalyst, with Runs 1, 3, and 5 not being activated, while Runs 2, 4 and 6 were activated in accordance with present invention. A comparison of Run 2 with Run 1 shows that by activating the catalyst, higher HCl conversions are possible at lower temperatures at the same EDC selectivity. Similar results are shown when Run 4 (activated) is compared with Run 3 (not activated), and Run 6 (activated) is compared with Run 5 (not activated). The data also shows the general trend that at lower copper loadings, higher temperatures are generally required to achieve the desired selectivity, but the advantages of the present invention are maintained in that activation of the catalyst allows for lower temperatures while achieving desired HCl conversions and EDC selectivity for each of the respective catalysts. Finally, the data show that activation can be achieved with or without oxygen in the feed stream during activation; Run 6 was activated with only a nitrogen feed and achieved results comparable to activation that occurred in the presence of a nitrogen and oxygen blend.

**Table I**

| | | | | 98% EDC Sel. | 98% EDC Sel. | 97% EDC Sel. | 97% EDC Sel. | Activation | Activator | Activation Time (Hours) |
|---|---|---|---|---|---|---|---|---|---|---|
| Run | Cu (wt%) | Mg (wt%) | Zr (wt%) | Temp. (°C) | HCl Conv. (%) | Temp. (°C) | HCl Conv. (%) | No/Yes - Temp. (°C) | N₂ (%) / O₂(%) | |
| 1 | 5.0 | 1.30 | 1.50 | 210.3 | 99.2 | 215.0 | 99.4 | No | N/A | N/A |
| 2 | 5.0 | 1.30 | 1.50 | 207.9 | 99.5 | 212.6 | 99.6 | Yes - 225 | 79/21 | 88 |
| 3 | 4.7 | 1.80 | 0.95 | 215.0 | 98.8 | 219.1 | 99.0 | No | N/A | N/A |
| 4 | 4.7 | 1.80 | 0.95 | 211.1 | 99.2 | 215.4 | 99.4 | Yes - 230 | 88/12 | 64 |
| 5 | 4.5 | 1.90 | 0.95 | 211.4 | 98.7 | 215.8 | 99.0 | No | N/A | N/A |
| 6 | 4.5 | 1.90 | 0.95 | 210.5 | 98.9 | 215.0 | 99.2 | Yes - 230 | 100/0 | 64 |

### Data Set II - Runs 7-8

A second data set, which is summarized in Table II below, shows that the run time of the reactor does not achieve the same results as catalyst activation. Namely, data was collected for Run 7 after the reactor ran (under oxychlorination conditions) for about 50 days at various temperatures (together with about 16 sporadic days of room temperature fluidization with nitrogen while HCl and ethylene feeds were turned off). Despite having run for this extended period of time, Run 7 did not achieve the benefits observed with Run 8, which was activated in accordance with the present invention; again, as evidenced by achieving higher HCl conversion at targeted EDC selectivity at lower temperatures.

**Table II**

| | | | | 98% EDC Sel. | 98% EDC Sel. | 97% EDC Sel. | 97% EDC Sel. | Activation | Activator | Activation Time (Hours) |
|---|---|---|---|---|---|---|---|---|---|---|
| Run | Cu (wt%) | Mg (wt%) | Zr (wt%) | Temp. (°C) | HCl Conv. (%) | Temp. (°C) | HCl Conv. (%) | No/Yes - Temp. (°C) | N₂ (%) / O₂(%) | |
| 7 | 4.5 | 1.90 | 0.95 | 209.8 | 98.7 | 214.6 | 99.0 | No | N/A | N/A |
| 8 | 4.5 | 1.90 | 0.95 | 208.8 | 99.0 | 213.8 | 99.2 | Yes - 230 | 88/12 | 64 |

### Data Set III - Runs 9-12

A third data set, which is summarized in Table III below, shows that catalyst activation is a function of time and temperature. Each run represents a data set collected from the same reactor in succession (i.e. after the reactor ran and data was collected for the previous run). Specifically, for Run 9, the catalyst was fluidized with the nitrogen/oxygen blend for about 64 hours at 200 °C, and then the oxygen, ethylene, and HCl streams were started, and oxychlorination took place for about 104 hours. The oxygen, ethylene, and HCl feeds were then turned off and the catalyst was fluidized with a nitrogen/oxygen blend for another 64 hours at 200 °C. The oxygen, ethylene, and HCl feeds were again turned on after about 64 hours, and oxychlorination continued for approximately another 104 hours, which represents Run 10. The same pattern was followed for Runs 11 and 12, except that the catalyst was heated to 230 °C for 64 hours under fluidization for Run 12. A comparison of Run 12 with Run 9 shows that heating the catalyst at 200 °C for 64 hours did not activate the catalyst to the same degree as longer times at 200 °C (see Runs # 10 and 11) or at higher temperatures (see Run 12). It should nonetheless be noted that Runs 10 and 11 show the trend that activation at 200 °C eventually improves catalyst performance as activation time is extended. Runs 9-11 are not according to the invention but for reference only.

**Table III**

| | | | | 98% EDC Sel. | 98% EDC Sel. | 97% EDC Sel. | 97% EDC Sel. | Activation | Activator | Activation Time (Hours) |
|---|---|---|---|---|---|---|---|---|---|---|
| Run | Cu (wt%) | Mg (wt%) | Zr (wt%) | Temp. (°C) | HCl Conv. (%) | Temp. (°C) | HCl Conv. (%) | No/Yes - Temp. (°C) | N₂ (%) / O₂(%) | |
| 9 | 4.5 | 1.90 | 0.95 | 214.8 | 98.9 | 219.4 | 99.1 | Yes - 200 | 88/12 | 64 |
| 10 | 4.5 | 1.90 | 0.95 | 213.8 | 98.9 | 218.5 | 99.2 | Yes - 200 | 88/12 | 128 |
| 11 | 4.5 | 1.90 | 0.95 | 213.3 | 99.0 | 218.1 | 99.3 | Yes - 200 | 88/12 | 192 |
| 12 | 4.5 | 1.90 | 0.95 | 211.4 | 99.1 | 216.4 | 99.3 | Yes - 230 | 88/12 | 256 |

### Data Set IV - Runs 13-16

A fourth data set, which is summarized in Table IV below, shows that the catalyst must be activated within the reactor under fluidization conditions. The catalysts employed in Runs 14 and 16 were activated in accordance with the invention, while an attempt was made to activate the catalyst employed in Runs 13 and 15 within an oven at 230 °C for 64 hours. The catalyst employed in Run 13 was cooled to 110 °C for 24 hours before being loaded into the reactor, while the catalyst employed in Run 15 was transferred directly from the oven to the reactor. As the data indicates, neither of the catalysts that were heated in the oven (Runs 13 and 15) were activated in the same way as the catalysts activated by the present invention (Runs 14 and 16).

**Table IV**

| | | | | 98% EDC Sel. | 98% EDC Sel. | 97% EDC Sel. | 97% EDC Sel. | Activation | Activator | Activation Time (Hours) |
|---|---|---|---|---|---|---|---|---|---|---|
| Run | Cu (wt%) | Mg (wt%) | Zr (wt%) | Temp. (°C) | HCl Conv. (%) | Temp. (°C) | HCl Conv. (%) | No/Yes - Temp. (°C) | N₂ (%) / O₂(%) | |
| 13 | 4.5 | 1.90 | 0.95 | 212.9 | 98.8 | 217.7 | 99.1 | Oven/Air | N/A | N/A |
| 14 | 4.5 | 1.90 | 0.95 | 209.8 | 99.2 | 214.5 | 99.4 | Yes - 230 | 88/12 | 64 |
| 15 | 4.5 | 1.90 | 0.95 | 216.8 | 98.7 | 221.4 | 99.1 | Oven/Air | N/A | N/A |
| 16 | 4.5 | 1.90 | 0.95 | 214.8 | 99.1 | 219.1 | 99.3 | Yes - 230 | 88/12 | 64 |

### Data Set V - Runs 17-20

A fifth data set, which is summarized in Table V below, shows that the discoveries associated with the present invention extend to other catalyst systems. For example, catalysts that employ potassium and rare earth metals, which are known catalyst systems for oxychlorination, benefit from practice of the present invention. One catalyst used in this experiment (Runs # 17 & #18) included 1.5 wt % lanthanum, 0.5 wt % cerium, and 0.5 wt % praseodymium (represented as 2.5 wt % rare earth in Table V). As the data suggests, activation of the catalyst according to the invention (i.e. Run #18) provided for higher HCl conversions at lower temperatures at the same EDC selectivity as compared to the same system but without activation (i.e. Run #17). Similar results are shown for catalysts that include copper and magnesium (Runs #19 and #20).

**Table V**

| | | | | Other (wt%) | 98% EDC Sel. | 98% EDC Sel. | 97% EDC Sel. | 97% EDC Sel. | Activation | Activator | Activation Time (Hours) |
|---|---|---|---|---|---|---|---|---|---|---|---|
| Run | Cu (wt%) | Mg (wt%) | K (wt%) | Rare Earth (wt %) | Temp. (°C) | HCl Conv. (%) | Temp. (°C) | HCl Conv. (%) | No/Yes - Temp. (°C) | N₂ (%) / O₂(%) | |
| 17 | 4.3 | 1.30 | 1.10 | 2.50 | 220.8 | 99.2 | 226.6 | 99.4 | No | N/A | N/A |
| 18 | 4.3 | 1.30 | 1.10 | 2.50 | 218.5 | 99.3 | 224.2 | 99.5 | Yes - 230 | 88/12 | 64 |
| 19 | 4.15 | 2.12 | N/A | N/A | 222.8 | 97.8 | 226.9 | 98.5 | No | N/A | N/A |
| 20 | 4.15 | 2.12 | N/A | N/A | 219.1 | 99.1 | 224.4 | 99.3 | Yes - 235 | 88/12 | 64 |

## Claims

1. A method for the production of 1,2-dichloroethane, the method comprising:
(i) providing a fluidizable catalyst;
(ii) charging the fluidizable catalyst to a fluidized bed reactor;
(iii) fluidizing the catalyst within the reactor;
(iv) heating the catalyst to a temperature of greater than 210 °C for greater than 24 hours within the fluidized bed reactor while fluidizing the catalyst within the reactor, where said steps of fluidizing and heating take place in the absence of the conversion of ethylene; and
(v) after said step of heating, converting ethylene to 1,2-dichloroethane in the presence of the catalyst.

2. The method of claim 1, where said step of heating takes place for greater than 36 hours.

3. The method of any of the preceding claims, further comprising the step of feeding oxygen to the reactor.

4. The method of any of the preceding claims, where said step of converting ethylene to 1,2-dichloroethane includes the step of feeding ethylene to the reactor.

5. The method of any of the preceding claims, where said step of converting ethylene to 1,2-dichloroethane includes the step of feeding hydrochloric acid to the reactor.

6. The method of any of the preceding claims, where said steps of heating and fluidizing takes place in the substantial absence of hydrochloric acid.

7. The method of any of the preceding claims, where said step of heating takes place in the presence of oxygen, and/or an oxygen-containing gas, and/or inert gas.

8. The method of any of the preceding claims, where the fluidizable catalyst includes a support impregnated with a catalytic metal species.

9. The method of any of the preceding claims, where the catalytic metal species includes copper.

10. The method of any of the preceding claims, where the catalytic metal species includes copper, magnesium and zirconium.

11. The method of any of the preceding claims, where the ethylene contacts the catalyst within a reaction zone of the reactor.

12. The method of any of the preceding claims, further comprising the step of recovering 1,2-dichloroethane from an effluent stream of the reactor.

13. The method of any of the preceding claims, where the support is a high-surface area alumina.

## Patentansprüche

1. Verfahren zum Herstellen von 1,2-Dichlorethan, das Verfahren umfassend:
(i) Bereitstellen eines fluidisierbaren Katalysators;
(ii) Einbringen des fluidisierbaren Katalysators in einen Wirbelschichtreaktor;
(iii) Fluidisieren des Katalysators in dem Reaktor;
(iv) Erhitzen des Katalysators auf eine Temperatur von mehr als 210 °C für mehr als 24 Stunden in dem Wirbelschichtreaktor unter Fluidisieren des Katalysators in dem Reaktor, wobei die Schritte des Fluidisierens und Erhitzens in Abwesenheit der Umwandlung von Ethylen stattfinden; und
(v) nach dem Schritt des Erhitzens, Umwandeln von Ethylen in 1,2-Dichlorethan in Gegenwart des Katalysators.

2. Verfahren nach Anspruch 1, wobei der Schritt des Erhitzens für mehr als 36 Stunden erfolgt.

3. Verfahren nach einem der vorstehenden Ansprüche, ferner umfassend den Schritt des Zuführens von Sauerstoff zu dem Reaktor.

4. Verfahren nach einem der vorstehenden Ansprüche, wobei der Schritt des Umwandelns von Ethylen in 1,2-Dichlorethan den Schritt des Zuführens von Ethylen zu dem Reaktor einschließt.

5. Verfahren nach einem der vorstehenden Ansprüche, wobei der Schritt des Umwandelns von Ethylen in 1,2-Dichlorethan den Schritt des Zuführens von Salzsäure zu dem Reaktor einschließt.

6. Verfahren nach einem der vorstehenden Ansprüche, wobei die Schritte des Erhitzens und Fluidisierens in wesentlicher Abwesenheit von Salzsäure stattfinden.

7. Verfahren nach einem der vorstehenden Ansprüche, wobei der Schritt des Erhitzens in Gegenwart von Sauerstoff und/oder einem sauerstoffhaltigen Gas und/oder Inertgas stattfindet.

8. Verfahren nach einem der vorstehenden Ansprüche, wobei der fluidisierbare Katalysator einen mit einer katalytischen Metallspezies imprägnierten Träger einschließt.

9. Verfahren nach einem der vorstehenden Ansprüche, wobei die katalytische Metalle Spezies Kupfer einschließt.

10. Verfahren nach einem der vorstehenden Ansprüche, wobei die katalytische Metalle Spezies Kupfer, Magnesium und Zirkonium einschließt.

11. Verfahren nach einem der vorstehenden Ansprüche, wobei das Ethylen den Katalysator innerhalb einer Reaktionszone des Reaktors kontaktiert.

12. Verfahren nach einem der vorstehenden Ansprüche, ferner umfassend den Schritt des Rückgewinnens von 1,2-Dichlorethan aus einem Abflussstrom des Reaktors.

13. Verfahren nach einem der vorstehenden Ansprüche, wobei der Träger eine Tonerde mit hohen/ großen Oberfläche ist.

## Revendications

1. Procédé pour la production de 1,2-dichloroéthane, le procédé comprenant :
(i) la fourniture d'un catalyseur fluidisable ;
(ii) le chargement du catalyseur fluidisable dans un réacteur à lit fluidisé ;
(iii) le fait de fluidiser le catalyseur au sein du réacteur ;
(iv) le chauffage du catalyseur à une température supérieure à 210 °C pendant plus de 24 heures au sein du réacteur à lit fluidisé tout en fluidisant le catalyseur au sein du réacteur, où lesdites étapes de fluidisation et de chauffage ont lieu en l'absence de la conversion d'éthylène ; et
(v) après ladite étape de chauffage, la conversion d'éthylène en 1,2-dichloroéthane en présence du catalyseur.

2. Procédé selon la revendication 1, où ladite étape de chauffage a lieu pendant plus de 36 heures.

3. Procédé selon l'une quelconque des revendications précédentes, comprenant en outre l'étape consistant à alimenter le réacteur en oxygène.

4. Procédé selon l'une quelconque des revendications précédentes, où ladite étape de conversion de l'éthylène en 1,2-dichloroéthane comprend l'étape d'alimentation du réacteur en éthylène.

5. Procédé selon l'une quelconque des revendications précédentes, où ladite étape de conversion d'éthylène en 1,2-dichloroéthane comprend l'étape d'alimentation du réacteur en acide chlorhydrique.

6. Procédé selon l'une quelconque des revendications précédentes, où lesdites étapes de chauffage et de fluidisation ont lieu en l'absence substantielle d'acide chlorhydrique.

7. Procédé selon l'une quelconque des revendications précédentes, où ladite étape de chauffage a lieu en présence d'oxygène, et/ou d'un gaz contenant de l'oxygène, et/ou de gaz inerte.

8. Procédé selon l'une quelconque des revendications précédentes, où le catalyseur fluidisable comporte un support imprégné d'une espèce métallique catalytique.

9. Procédé selon l'une quelconque des revendications précédentes, dans lequel l'espèce métallique catalytique comporte du cuivre.

10. Procédé selon l'une quelconque des revendications précédentes, dans lequel l'espèce métallique catalytique comporte du cuivre, du magnésium et du zirconium.

11. Procédé selon l'une quelconque des revendications précédentes, où l'éthylène entre en contact avec le catalyseur au sein d'une zone de réaction du réacteur.

12. Procédé selon l'une quelconque des revendications précédentes, comprenant en outre l'étape consistant à récupérer du 1,2-dichloroéthane à partir d'un courant d'effluent du réacteur.

13. Procédé selon l'une quelconque des revendications précédentes, où le support est une alumine à surface spécifique élevée.
